# EUROPEAN PATENT APPLICATION

(11) **EP 4 190 904 A1**
(43) Date of publication of application: **07.06.2023**
(21) Application number: 21864631.3
(22) Date of filing: 01.09.2021
(51) Int. Cl.: C12N 15/77, C12N 9/88, C12N 9/10, C12N 9/02, C12P 13/08

(54) **L-VALINE-PRODUCING MICROORGANISMS AND L-VALINE PRODUCING METHOD USING SAME**

(30) Priority: 01.09.2020 KR 20200111084
(71) Applicant: CJ Cheiljedang Corporation, Seoul 04560 (KR)
(72) Inventor: CHANG, Jin Sook, Seoul 04560 (KR); KIM, Seon Hye, Seoul 04560 (KR); YOON, Byoung Hoon, Seoul 04560 (KR); KIM, Ju-yeon, Seoul 04560 (KR); KIM, Hyung Joon, Seoul 04560 (KR); CHOI, Sun Hyoung, Seoul 04560 (KR)
(74) Representative: Heubeck, Christian
(86) International application number: PCT/KR2021/011717
(87) International publication number: WO 2022/050671

(57) **Abstract**

The present disclosure relates to a microorganism producing L-valine and a method for producing L-valine using the microorganism, and when the microorganism comprising a combination of enzymes having enhanced or reduced activity of the present disclosure is cultured, L-valine can be produced in high yield.

## Description

### [Technical Field]

The present disclosure relates to a microorganism producing L-valine and a method for producing L-valine using the microorganism.

### [Background Art]

L-Valine, a branched-chain amino acid, is biosynthesized in microorganisms, starting from pyruvic acid via acetolactic acid, dihydroxy isovaleric acid, and ketoisovaleric acid. These intermediate metabolites are produced by a reaction catalyzed by acetohydroxy acid synthase, acetohydroxy acid isomeroreductase, dihydroxy acid dehydratase, and transaminase B. However, these enzymes are also involved in L-isoleucine biosynthesis starting from ketobutyric acid and pyruvic acid, and L-leucine is also biosynthesized from the intermediate metabolite, ketoisovaleric acid, via 2-isopropylmalic acid, 3-isopropylmalic acid, and ketoisocaproic acid. Therefore, since the enzymes used in the biosynthetic pathways of the branched-chain amino acids, *i.e*., L-valine, L-isoleucine, and L-leucine, are identical, it is known that industrially producing one type of branched-chain amino acid through fermentation is difficult. Additionally, feedback inhibition occurs by the final product L-valine or derivatives thereof, which makes it difficult for industrial mass production of L-valine.

However, there have thus far been studies on a production method of valine through feedback inhibition (US Patent No. 10457919), but there have not been any studies on the increase in valine-producing ability through the combination of enzymes with enhanced or reduced activity of the present disclosure.

### [Disclosure]

### [Technical Problem]

Accordingly, the present inventors have conducted continuous research on an effective method for producing L-valine, and as a result, it was confirmed that the microorganism including enhanced dihydroxy-acid dehydratase activity; reduced transaminase C activity; weakened pyruvate dehydrogenase activity; reduced citrate synthase activity; or a combination thereof has a superior L-valine-producing ability as compared to a wild-type microorganism, thereby completing the present invention.

### [Technical Solution]

It is one object of the present disclosure to provide a microorganism producing L-valine having enhanced dihydroxy-acid dehydratase activity; and any one or more combinations selected from (1) to (3) below:
(1) reduced transaminase C activity;
(2) weakened pyruvate dehydrogenase activity;
(3) reduced citrate synthase activity.

It is another object of the present disclosure to provide a method for producing L-valine, including culturing the microorganism.

### [Advantageous Effects]

When culturing the microorganism producing L-valine of the present disclosure, L-valine can be produced in high yield. Accordingly, the effects of production convenience and production cost reduction can be expected in terms of the industrial aspect.

### [Detailed Description of Preferred Embodiments]

Hereinafter, the present disclosure will be described in detail.

Meanwhile, each description and embodiment disclosed herein can be applied to other descriptions and embodiments with respect to common features. That is, all combinations of various elements disclosed herein fall within the scope of the present disclosure. Further, the scope of the present disclosure is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present disclosure.

One aspect of the present disclosure provides a microorganism producing L-valine having enhanced dihydroxy-acid dehydratase activity; and any one or more combinations selected from (1) to (3) below:
(1) reduced transaminase C activity;
(2) weakened pyruvate dehydrogenase activity; and
(3) reduced citrate synthase activity.

In one embodiment, the microorganism producing valine may be a microorganism having enhanced dihydroxy-acid dehydratase activity and reduced transaminase C activity.

In another embodiment, the microorganism producing valine may be a microorganism having enhanced dihydroxy-acid dehydratase activity and weakened pyruvate dehydrogenase activity.

In still another embodiment, the microorganism producing valine may be a microorganism having enhanced dihydroxy-acid dehydratase activity and reduced citrate synthase activity.

In yet another embodiment, the microorganism producing valine may be a microorganism having enhanced dihydroxy-acid dehydratase activity and reduced citrate synthase activity, and additionally having reduced transaminase C activity or weakened pyruvate dehydrogenase activity, but is not limited thereto.

As used herein, the term "dihydroxy-acid dehydratase" is an enzyme involved in the synthesis of ketoiso-valerate, a precursor of valine, in the biosynthetic pathway of producing L-valine starting from pyruvate via acetolactate, dihydroxy-isovalerate to ketoiso-valerate. In the present disclosure, the production of L-valine can be promoted by enhancing the activity of dihydroxy-acid dehydratase, and thus increasing the synthesis of ketoiso-valerate.

As used herein, the term "transaminase C" is an enzyme involved in the pathway for synthesizing L-alanine from pyruvate.

As used herein, the term "pyruvate dehydrogenase" is an enzyme involved in the synthesis of acetyl-coA from pyruvic acid.

As used herein, the term "citrate synthase" is an enzyme synthesizes citrate from acetyl-coA.

As used herein, the term "enhancement" means that the activity of a protein is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the dihydroxy-acid dehydratase can be confirmed by the increase in the level of activity of the polypeptide, expression level, or the amount of product excreted from the polypeptide.

The enhancement of the activity of the dihydroxy-acid dehydratase can be applied by various methods well known in the art, and the method is not limited as long as it can enhance the activity of the target polypeptide compared to that of a microorganism before modification. Specifically, genetic engineering and/or protein engineering well known to those skilled in the art, which is a common method of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, *etc*.).

Specifically, the enhancement of the activity of the dihydroxy-acid dehydratase of the present disclosure may be achieved by:
1) increasing the intracellular copy number of a polynucleotide encoding the polypeptide;
2) replacing the expression regulatory region of a gene encoding the polypeptide on the chromosome with a sequence having a stronger activity;
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

### More specifically,

The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, the method may include replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.*, but the strong promoter is not limited thereto.

The 3) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

Further, the 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

The vector of the present disclosure is a DNA molecule used as a mediator for artificially transporting foreign genetic materials to other cells and may include a DNA construct containing a nucleotide sequence of a polynucleotide encoding a target polypeptide operably linked to a suitable expression regulatory region (expression regulatory sequence) so as to be able to express the target polypeptide in a suitable host cell. The expression regulatory region may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptII, pGEM, pTZ, pCL and pET, *etc.* may be used. Specifically, pDZ, pDC, pDCM2, pACYC177, pACYC184, pCL, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the gene sequence is functionally linked to a promoter sequence that initiates and mediates transcription of the polynucleotide encoding the target protein of the present disclosure.

Such enhancement of the protein activity may mean that the activity of the corresponding protein not originally possessed is exhibited, or the activity or concentration thereof is generally increased by 1 %, 10 %, 25 %, 50 %, 75 %, 100 %, 150 %, 200 %, 300 %, 400 % or 500 %, and maximum of 1000 % or 2000 % or more based on the activity or concentration of a wild-type protein or an initial microbial strain, but is not limited thereto.

Specifically, the enhancement of dihydroxy-acid dehydratase activity may mean that the dihydroxy-acid dehydratase activity in a microorganism is enhanced compared to that of a wild-type microorganism, a microorganism before modification or a microorganism having non-modified protein, thereby increasing the synthesis of ketoiso-valerate, which is a precursor of L-valine, from dihydroxy-isovalaerate, resulting in an increase in L-valine production.

As used herein, the term "reduction" is a comprehensive concept including the case where the activity of a protein exhibited in a microorganism in its natural state or before modification is weakened or removed (deleted), i.e., when compared to the endogenous activity or one copy of a gene encoding the protein in a cell, and may mean that the activity is 0% or more to 100% or less.

Such "reduction of activity" of a protein means that the activity of the protein itself is removed or an effect less than its original function is realized, but is not particularly limited thereto. That is, the reduction of activity specifically includes both "deletion of activity" and "weakening of activity".

The "deletion of activity" may mean that an enzyme or protein is not expressed at all compared to a natural wild-type strain, a parent strain or a strain having non-modified protein, or the activity thereof is not observed even when the enzyme or protein is expressed.

In the present disclosure, the deletion of the activity may be achieved by applying various methods well known in the art. Examples of the methods include: 1) deleting a part or all of the gene encoding the protein; 2) modifying the gene sequence encoding the protein such that the protein activity is removed or weakened; 3) introducing an antisense oligonucleotide (e.g., antisense RNA), which binds complementary to the gene transcript encoding the protein; 4) adding a sequence complementary to the Shine-Dalgarno (SD) sequence on the front end of the SD sequence of the gene encoding the protein to form a secondary structure, thereby inhibiting the ribosomal attachment; 5) a reverse transcription engineering (RTE), which adds a promoter, which is to be reversely transcribed, on the 3' terminus of the open reading frame (ORF) of the gene sequence encoding the protein; or a combination thereof, but are not particularly limited thereto.

Additionally, the "weakening of activity" may mean that an effect less than its original function is realized, and may be achieved by methods including: deleting a part of a gene encoding the protein on the chromosome; replacing the gene encoding the protein on the chromosome with a mutated gene such that the activity of the protein is reduced; introducing a mutation into an expression regulatory sequence of the gene encoding the protein on the chromosome; replacing the expression regulatory sequence of the gene encoding the protein with a sequence having weak activity (e.g., replacing the promoter of the gene with a promoter weaker than the endogenous promoter), *etc.,* but is not limited thereto, and known methods for weakening of activity can be used without limitation.

Such reduction of protein activity may mean that the activity of the corresponding protein is removed, or the activity or concentration thereof is generally reduced by 0%, 1 %, 5%, 10 %, 15%, 20%, 30%, 40%, 50%, 60%, 70%, 80%, 90%, 95%, 98%, 99% based on the activity or concentration in a wild-type protein or an initial microbial strain, but is not limited thereto.

Specifically, the activity of transaminase C in the microorganism may be reduced compared to that of a natural wild-type strain, a parent strain before mutation, or a strain having non-modified protein. In one embodiment, the microorganism may have no activity of transaminase C protein due to the deletion of transaminase C gene, or may have weakened activity as the start codon coding sequence of the transaminase C gene is modified to GTG, thereby reducing the expression of the transaminase C protein.

Additionally, specifically, the activity of pyruvate dehydrogenase in the microorganism may be reduced compared to that of a natural wild-type strain, a parent strain before mutation, or a strain having non-modified protein. In one embodiment, the microorganism may have no activity of pyruvate dehydrogenase protein due to the deletion of pyruvate dehydrogenase gene, or may have weakened activity as the start codon coding sequence of the pyruvate dehydrogenase gene is modified to GTG, thereby reducing the expression of the pyruvate dehydrogenase protein. In another embodiment, as the sequence of the pyruvate dehydrogenase gene is mutated in the microorganism, the microorganism may express a pyruvate dehydrogenase mutant having weakened activity than that of the wild-type protein, in which the amino acid corresponding to the 432^{nd} or 435^{th} position from the N-terminus of the amino acid sequence of SEQ ID NO: 3 is substituted with another amino acid. In particular, the pyruvate dehydrogenase mutant having weakened activity than that of the wild-type protein may include the sequence of SEQ ID NO: 5 or SEQ ID NO: 6, but is not limited thereto.

Further, specifically, the activity of citrate synthase in the microorganism may be reduced compared to that of a natural wild-type strain, a parent strain before mutation, or a strain having non-modified protein. In one embodiment, the microorganism may have no activity of citrate synthase protein due to the deletion of citrate synthase gene, or may have weakened activity as the start codon coding sequence of the citrate synthase gene is modified to GTG, thereby reducing the expression of the citrate synthase protein. In another embodiment, as the sequence of the citrate synthase gene is mutated in the microorganism, the microorganism may express a citrate synthase mutant having reduced activity than that of the wild-type protein, in which the amino acid corresponding to the 241^{st} or 312^{nd} position from the N-terminus of the amino acid sequence of SEQ ID NO: 4 is substituted with another amino acid. In particular, the citrate synthase mutant having reduced activity than that of the wild-type protein may include the sequence of SEQ ID NO: 7 or SEQ ID NO: 8, but is not limited thereto.

The expression 'substitution with another amino acid' is not limited as long as an amino acid before substitution is substituted with another amino acid. Specifically, it may be substituted with any one amino acid selected from lysine, histidine, glutamic acid, aspartic acid, glycine, alanine, valine, leucine, isoleucine, methionine, phenylalanine, tryptophan, proline, serine, threonine, cysteine, tyrosine, asparagine, arginine and glutamine

More specifically, the pyruvate dehydrogenase having weakened activity may be one in which the amino acid corresponding to the 432^{nd} or 435^{th} position from the N-terminus of the amino acid sequence of SEQ ID NO: 3 with a non-polar amino acid.

Additionally, more specifically, the citrate synthase having reduced activity may be one in which the amino acid corresponding to the 241^{st} or 312^{nd} position from the N-terminus of the amino acid sequence of SEQ ID NO: 4 is substituted with a polar or non-polar amino acid.

As used herein, the term "corresponding to" refers to an amino acid residue at the position recited in a protein or peptide, or an amino acid residue which is similar, identical, or homologous to the residue recited in a protein or peptide. As used herein, the "corresponding region" generally refers to a similar or corresponding position in the related protein or reference protein.

In the present disclosure, a specific numbering of amino acid residue positions in the protein used herein may be employed. For example, it is possible to renumber the amino acid residue positions of the protein of the present disclosure to the corresponding positions by aligning the sequence of the protein of the present disclosure with the target protein to be compared.

As used herein, the term 'homology' or 'identity' refers to a degree of relevance between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277) (preferably, version 5.0.0 or versions thereafter) (GCG program package (Devereux, J., et al., Nucleic Acids Research 12: 387 (1984)), BLASTP, BLASTN, FASTA (Atschul, [S.] [F.,] [ET AL., J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48: 443 as disclosed in Smith and Waterman, Adv. Appl. Math (1981) 2:482. In summary, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

As used herein, the term "variant" refers to a protein having one or more amino acids different from the recited sequence by conservative substitutions and/or modifications such that the functions and properties of the protein are retained. The variants are different from the sequences identified by substitution, deletion or addition of several amino acids. Such variants may generally be identified by modifying one or more of the above amino acid sequences of the protein and evaluating the properties of the modified protein. That is, the ability of the variants may be enhanced, unchanged or reduced relative to a native protein. Other variants may include those in which a region has been removed from the N- and/or C-terminal of a mature protein. The term "variant" may be used interchangeably with terms such as modification, modified protein, modified polypeptide, mutant, mutein, divergent, variant, *etc*., as long as the terms are used to indicate variation, but the terms are not limited thereto. For the purpose of the present disclosure, the variant may be those in which the protein activity is reduced or weakened as compared to that of a natural wild-type protein or a non-modified protein, but is not limited thereto.

As used herein, the term "conservative substitution" refers to substitution of an amino acid with another amino acid having similar structural and/or chemical properties. Such amino acid substitution may generally occur based on similarity of polarity, charge (basic, acidic), solubility, hydrophobicity, hydrophilicity, and/or amphipathic nature of a residue.

Additionally, the variant may also include deletion or addition of amino acids that have minimal influence on the properties and secondary structure of a polypeptide. For example, the polypeptide may be conjugated with a signal (or leader) sequence at the N-terminal involved in the transfer of proteins co-translationally or post-translationally. Further, the polypeptide may also be conjugated with another sequence or linker to identify, purify, or synthesize the polypeptide.

As used herein, the term "microorganism producing L-valine" refers to a microorganism capable of producing an excess amount of L-valine from carbon source in a medium compared to a wild-type or non-modified microorganism. In addition, the microorganism producing L-valine may be a recombinant microorganism. Specifically, the microorganism is not particularly limited by its type as long as it can produce L-valine, and may be a microorganism of the genus *Enterobacter*, the genus *Escherichia*, the genus *Erwinia*, the genus *Serratia*, the genus *Providencia*, the genus *Corynebacterium,* and the genus *Brevibacterium*. More specifically, it may be a microorganism of the genus *Corynebacterium* or the genus *Escherichia.*

Even more specifically, the microorganism of the genus *Escherichia* may be *Escherichia coli*, and the microorganism of the genus *Corynebacterium* may be *Corynebacterium glutamicum*, and any microorganism of the genus *Corynebacterium* or the genus *Escherichia* capable of increasing L-valine production, in which enhanced dihydroxy-acid dehydratase activity; and any one or more combinations selected from reduced transaminase C activity; reduced pyruvate dehydrogenase activity; or reduced citrate synthase activity are introduced, may be included.

The parent strain of the microorganism producing L-valine, which is modified to have enhanced dihydroxy-acid dehydratase activity; and any one or more combinations selected from (1) to (3) below:
(1) reduced transaminase C activity;
(2) weakened pyruvate dehydrogenase activity; and
(3) reduced citrate synthase activity, is not particularly limited as long as it is a microorganism producing L-valine.

The microorganism producing L-valine may be a natural microorganism itself, or a microorganism having an improved L-valine-producing ability by inserting a gene related to an external L-valine production mechanism or enhancing or reducing (weakening or suppressing) the activity of an endogenous gene.

In one embodiment, the microorganism may be a microorganism producing L-valine having enhanced activity of dihydroxy-acid dehydratase compared to a wild-type or a parent strain before mutation, and reduced activity of transaminase C compared to a wild-type or a parental strain before mutation. In particular, the dihydroxy-acid dehydratase may be encoded by the ilvD gene, and the transaminase C may be encoded by the avtA gene. The genes may be derived from *Corynebacterium glutamicum*, but are not limited thereto.

In another embodiment, the microorganism may be a microorganism producing L-valine having enhanced activity of dihydroxy-acid dehydratase compared to a wild-type or a parent strain before mutation, and weakened activity of pyruvate dehydrogenase compared to a wild-type or a parent strain before mutation. In particular, the dihydroxy acid dehydratase may be encoded by the ilvD gene, and the pyruvate dehydrogenase may be encoded by the aceE gene. The genes may be derived from *Corynebacterium glutamicum*, but are not limited thereto.

In still another embodiment, the microorganism may be a microorganism producing L-valine having enhanced activity of dihydroxy-acid dehydratase compared to a wild-type or a parent strain before mutation, and reduced activity of citrate synthase compared to a wild-type or a parent strain before mutation. In particular, the dihydroxy acid dehydratase may be encoded by the ilvD gene, and the citrate synthase may be encoded by the gltA gene. The genes may be derived from *Corynebacterium glutamicum*, but are not limited thereto.

In addition to the above microorganism, it may be a microorganism producing L-valine in which the activity of transaminase C is reduced or the activity of pyruvate dehydrogenase is reduced compared to a wild-type or a parent strain before mutation, or may be a microorganism producing L-valine in which the activity of transaminase C is reduced and the activity of pyruvate dehydrogenase is reduced compared to a wild-type or a parent strain before mutation.

Another aspect of the present disclosure provides a method for producing L-valine, including: culturing a microorganism producing L-valine, which is characterized by having enhanced dihydroxy-acid dehydratase activity; and any one or more combinations selected from (1) to (3) below:
(1) reduced transaminase C activity;
(2) weakened pyruvate dehydrogenase activity; and
(3) reduced citrate synthase activity.

The "L-valine" of the present disclosure may include not only L-valine itself, but also a salt form thereof.

As used herein, the term "cultivation" means that the microorganism is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and a fed-batch culture, but is not limited thereto.

As used herein, the term "medium" refers to a mixture of materials which contains nutrient materials required for the cultivation of the microorganism as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, while adjusting temperature, pH, *etc.*

In the present disclosure, the carbon source may include carbohydrates, such as glucose, fructose, sucrose, maltose, *etc*.; sugar alcohols, such as mannitol, sorbitol, *etc*.; organic acids, such as pyruvic acid, lactic acid, citric acid, *etc*.; amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e*., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc*.; amino acids, such as glutamic acid, methionine, glutamine, *etc*.; and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors may be included. These constituting ingredients or precursors may be added to a medium in a batch or continuous manner, but these phosphorus sources are not limited thereto.

In the present disclosure, the pH of the medium may be adjusted by adding a compound such as ammonium hydroxide, potassium hydroxide, ammonia, phosphoric acid, sulfuric acid, *etc.* during the cultivation of the microorganism in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature of the medium may be in the range from 20°C to 50°C, specifically from 30°C to 37°C, but is not limited thereto. The cultivation may be carried out until a desired production amount of useful materials is obtained, specifically for about 10 to 100 hours, but is not limited thereto.

The L-valine produced by the cultivation of the present disclosure may be released into the medium or remain in the cells.

The method for producing L-valine of the present disclosure may further include a step of preparing the microorganism of the present disclosure, a step of preparing a medium for culturing the strain, or a combination thereof (regardless of the order, in any order), for example, prior to the culturing step.

The method for producing L-valine of the present disclosure may further include a step of recovering L-valine from the culture medium (medium on which the culture was grown) or the microorganism of the present disclosure. The recovering step may be further included after the culturing step.

In the recovering step, the desired L-valine may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc*., HPLC or a combination thereof may be used, and the desired L-valine can be recovered from the medium or the microorganisms using suitable methods known in the art.

Further, the method for producing L-valine of the present disclosure may further include a purification step, which may be performed using an appropriate method known in the art. In one example, when the method for producing L-valine of the present disclosure includes both a recovering step and a purification step, the recovering step and the purification step may be performed continuously or intermittently regardless of the order or simultaneously, or may be integrated into one step, but the method is not limited thereto.

In the method of the present disclosure, the polynucleotide, vector, microorganism, L-valine *etc*., are as described in the other aspects above.

Still another aspect of the present disclosure provides a method for increasing L-valine-producing ability, including modifying into a microorganism, which is characterized by having enhanced dihydroxy-acid dehydratase activity; and any one or more combinations selected from (1) to (3) below:
(1) reduced transaminase C activity;
(2) weakened pyruvate dehydrogenase activity; and
(3) reduced citrate synthase activity.

Yet another aspect of the present disclosure provides the use of a microorganism having enhanced dihydroxy-acid dehydratase activity; and any one or more combinations selected from (1) to (3) below:
(1) reduced transaminase C activity;
(2) weakened pyruvate dehydrogenase activity; and
(3) reduced citrate synthase activity, in the production of L-valine.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples.

### Example 1: Construction of Valine Production-Based Strain and Evaluation Thereof

One type of mutation [ilvN(A42V); Biotechnology and Bioprocess Engineering, June 2014, Volume 19, Issue 3, pp 456-467] was introduced into the wild-type *Corynebacterium glutamicum* ATCC14067 and ATCC13869 strains to construct a strain having an enhanced L-valine-producing ability.

Specifically, the genomic DNA of the wild-type *Corynebacterium glutamicum* ATCC14067 strain was extracted using a G-spin Total DNA extraction mini kit (intron, Cat. No 17045) according to the protocol provided in the kit. PCR was performed using a primer pair of SEQ ID NOS: 9 and 10 and a primer pair of SEQ ID NOS: 11 and 12 based on the genomic DNA as a template to obtain a gene fragment of 537 bp, respectively. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes.

Overlapping PCR was performed based on the two fragments obtained above as a template using a primer pair of SEQ ID NOS: 9 and 12 to obtain a PCR product of 1044 bp (hereinafter referred to as "mutation-introduced fragment 2").

The thus-obtained mutation-introduced fragment 2 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct a vector containing the mutation-introduced fragment 2. The vector for introducing A42V mutation into the ilvN gene was named pDZ-ilvN(A42V).

**[Table 1]**

| Primers | Nucleotide Sequences | SEQ ID NO: |
|---|---|---|
| Primer 1 | AATTTCTAGAGGCAGACCCTATTCTATGAA GG | 9 |
| Primer 2 | AGTGTTTCGGTCTTTACAGACACGAGGGA C | 10 |
| Primer 3 | GTCCCTCGTGTCTGTAAAGACCGAAACAC T | 11 |
| Primer 4 | AATTTCTAGACGTGGGAGTGTCACTCGCT TGG | 12 |

Thereafter, the pDZ-ilvN(A42V) was transformed into each of the wild-type *Corynebacterium glutamicum* ATCC14067 and ATCC13869 strains to induce homologous recombination on the chromosome (van der Rest et al., Appl Microbiol Biotechnol 52:541-545, 1999). The strains introduced with the vector on the chromosome by recombination of homologous sequences were selected in a medium containing 25 mg/L of kanamycin.

Subsequently, the gene fragments were amplified based on the *Corynebacterium glutamicum* transformants selected above by PCR using a primer pair of SEQ ID NOS: 18 and 21, and the introduction of the mutation was confirmed by sequencing analysis. The recombinant strains were named *Corynebacterium glutamicum* CJ7V and CJ8V, respectively.

The experiment on fermentation titer was carried out based on the wild-type *Corynebacterium glutamicum* ATCC14067 and ATCC13869, and the CJ7V and CJ8V strains constructed above. Each strain was sub-cultured in a nutrient medium, and then seeded into a 250 ml corner-baffle flask containing 25 ml of a production medium and cultured at 30°C for 72 hours at 200 rpm under shaking. Thereafter, the concentration of L-valine was analyzed using HPLC, and the analyzed concentration of L-valine is shown in Table 2 below.

### <Nutrient Medium (pH 7.2)>

Glucose 10 g, Meat Extract 5 g, Polypeptone 10 g, Sodium Chloride 2.5 g, Yeast Extract 5 g, Agar 20 g, Urea 2 g (based on 1 L of distilled water)

### <Production Medium (pH 7.0)>

Glucose 100 g, (NH₄)₂SO₄ 40 g, Soy Protein 2.5 g, Corn Steep Solids 5 g, Urea 3 g, KH₂PO₄ 1 g, MgSO₄.7H₂O 0.5 g, Biotin 100 µg, Thiamine-HCl 1 mg, Calcium-Pantothenic Acid 2 mg, Nicotinamide 3 mg, CaCOs 30 g (based on 1 L of distilled water)

**[Table 2]**

| Strains | OD600 | Valine (g/L) |
|---|---|---|
| ATCC14067 | 95 | 1.5 |
| CJ7V | 77 | 2.2 |
| ATCC13869 | 115 | 1.0 |
| CJ8V | 89 | 1.9 |

As shown in the results above, it was confirmed that the L-valine-producing ability was increased in the CJ7V and CJ8V strains introduced with ilvN(A42V) gene mutation as compared to the wild-type *Corynebacterium glutamicum* ATCC14067 and ATCC13869 strains.

### Example 2: Construction of Strains Having High Valine-Producing Ability and Evaluation Thereof

### Example 2-1. Construction of Valine Biosynthetic Gene ilvD-Enhanced Strains and Evaluation Thereof

The pDZ-Pcj7-ilvD vector for replacing the ilvD gene promoter was constructed using primer pairs of SEQ ID NOS: 13 and 14, SEQ ID NOS: 15 and 16, and SEQ ID NOS: 17 and 18 in order to construct a stain having high valine-producing ability, in which the expression of the valine biosynthetic gene ilvD was enhanced.

**[Table 3]**

| Primer Sequences for Constructing ilvD-Enhanced Vectors | | |
|---|---|---|
| Primers | Nucleotide Sequences | SEQ ID NO: |
| Primer 5 | TTCGAGCTCGGTACCCGGTCTAGAGCAC TTTCGCTCGCACC | 13 |
| Primer 6 | GATTTGAAAAGCGCATCAGAAACATCCC AGCGCTAC | 14 |
| Primer 7 | GTAGCGCTGGGATGTTTCTGATGCGCTT TTCAAATC | 15 |
| Primer 8 | GAAACACTATGATCCCACTTCGTTCAAAA GTCACCACCGTC | 16 |
| Primer 9 | GACGGTGGTGACTTTTGAACGAAGTGGG ATCATAGTGTTTC | 17 |
| Primer 10 | GCATGCCTGCAGGTCGACTCTAGAGCGT GTGCAACGCCGTC | 18 |

Specifically, in order to construct the pDZ-Pcj7-ilvD, PCR was performed using primer pairs of SEQ ID NOS: 13 and 14, SEQ ID NOS: 15 and 16, and SEQ ID NOS: 17 and 18 based on the genomic DNA of the wild-type *Corynebacterium glutamicum* ATCC14067 as a template to obtain a gene fragment, respectively. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes.

Overlapping PCR was performed based on the fragments obtained above as a template using a primer pair of SEQ ID NOS: 13 and 18 to obtain a mutation-introduced fragment 3. The thus-obtained mutation-introduced fragment 3 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct a vector, which was named pDZ-Pcj7-ilvD.

Thereafter, the pDZ-Pcj7-ilvD constructed above was transformed into each of the CJ7V, CJ8V, and KCCM11201P, which are valine production-based strains, to induce homologous recombination on the chromosome (van der Rest et al., Appl Microbiol Biotechnol 52:541-545, 1999). The strains introduced with the vector on the chromosome by recombination of homologous sequences were selected in a medium containing 25 mg/L of kanamycin. Subsequently, the gene fragments were amplified based on the *Corynebacterium glutamicum* transformants selected above by PCR using a primer pair of SEQ ID NOS: 13 and 18, and the introduction of the mutation was confirmed by gene sequencing analysis. The selected recombinant strains were named *Corynebacterium glutamicum* CJ7V:ilvD, CJ8V:ilvD, KCCM11201P:ilvD, respectively. The fermentation titer of the selected ilvD gene-enhanced strains was performed in the same manner as in Example 1 and the results are shown below.

**[Table 4]**

| L-Valine-Producing Ability of ilvD-Enhanced Strains | | |
|---|---|---|
| Strains | OD600 | Valine (g/L) |
| CJ7V | 77 | 2.2 |
| CJ7V:ilvD | 73 | 2.6 |
| CJ8V | 89 | 1.9 |
| CJ8V:ilvD | 88 | 2.2 |
| KCCM11201P | 62 | 2.6 |
| KCCM11201P:ilvD | 60 | 2.9 |

As shown in the results above, when ilvD, one of the valine biosynthetic genes, was enhanced, it was confirmed that the valine-producing ability of all valine-producing strains CJ7V, CJ8V and KCCM1 1201P was increased.

### Example 2-2. Construction of avtA-Deleted and a1g-Weakened Strains and Evaluation Thereof

In order to construct strains having high valine-producing ability, an attempt was made to weaken (modifying the start codon of avtA gene with GTG) or delete avtA. Accordingly, a vector for constructing an avtA-weakened strain (modifying the start codon of avtA gene with GTG) was constructed using primer pairs of SEQ ID NOS: 19 and 20, and SEQ ID NOS: 21 and 22 below, and a vector for constructing an avtA-deleted activity; using primer pairs of SEQ ID NOS: 23 and 24, and SEQ ID NOS: 25 and 26 below, which were named pDZ-avtA(A1g) and pDZ-avtA(del), respectively.

Specifically, in order to construct the avtA-weakened vector (pDZ-avtA(A1g)), PCR was performed using primer pairs of SEQ ID NOS: 19 and 20, and SEQ ID NOS: 21 and 22, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments obtained above as a template using a primer pair of SEQ ID NOS: 19 and 22 to obtain a mutation-introduced fragment 4. The thus-obtained mutation-introduced fragment 4 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-avtA(A1g) DNA vector.

In order to construct the avtA-deleted vector (pDZ-avtA(del)), PCR was performed using primer pairs of SEQ ID NOS: 23 and 24, and SEQ ID NOS: 25 and 26, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments obtained above as a template using a primer pair of SEQ ID NOS: 23 and 26 to obtain a mutation-introduced fragment 5. The thus-obtained mutation-introduced fragment 5 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-avtA(del) vector.

**[Table 5]**

| Primer Sequences for Constructing Vectors of avtA-Weakened or Deleted Strains | | |
|---|---|---|
| Primers | Nucleotide Sequences | SEQ ID NO: |
| Primer 11 | GCTCGGTACCCGGGGATCCTCTAGACCG CTTCCTTGGCTGCCTGAAGATG | 19 |
| Primer 12 | TGCTTGGCTTCACAAGAGACAAGCCT | 20 |
| Primer 13 | AGGCTTGTCTCTTGTGAAGCCAAGCA | 21 |
| Primer 14 | GCCTGCAGGTCGACCTAGATCTAGACCT CATCAGAGATAAGAACAGCATC | 22 |
| Primer 15 | GCTCGGTACCCGGGGATCCTCTAGATAC TCCGGTCTGCTTTATGCAGGTA | 23 |
| Primer 16 | AACTAACCTAGTCGCTTAAGAGACAAGC CTATCTGC | 24 |
| Primer 17 | GCAGATAGGCTTGTCTCTTAAGCGACTA GGTTAGTT | 25 |
| Primer 18 | GCCTGCAGGTCGACCTAGATCTAGAAAG TGCCACGAGCATTTCATCAGCT | 26 |

Thereafter, the pDZ-avtA(del) and pDZ-avtA(A1g) were transformed into each of the CJ7V:ilvD, CJ8V:ilvD, and KCCM11201P:ilvD, which are valine-producing strains, to induce homologous recombination on the chromosome (van der Rest et al., Appl Microbiol Biotechnol 52:541-545, 1999). The strains introduced with the vectors on the chromosome by recombination of homologous sequences were selected in a medium containing 25 mg/L of kanamycin.

Subsequently, the gene fragments were amplified based on the *Corynebacterium glutamicum* transformants selected above by PCR using primer pairs of SEQ ID NOS: 19 and 20, SEQ ID NOS: 21 and 22, SEQ ID NOS: 23 and 24, and SEQ ID NOS: 25 and 26, and the introduction of the mutation was confirmed by gene sequencing analysis in the same manner as in Example 1. The recombinant strains were named *Corynebacterium glutamicum* CJ7V:ilvD-avtA(del), CJ7V:ilvD-avtA (a1g), CJ8V:ilvD-avtA(del), CJ8V:ilvD-avtA (a1g), KCCM11201P:ilvD-avtA(del), and KCCM11201P:ilvD-avtA (a1g) as shown below, and the titer evaluation was performed in the same manner as in Example 1.

**[Table 6]**

| L-Valine-Producing Ability of avtA-Deleted and Weakened Strains | | |
|---|---|---|
| Strains | OD600 | Valine (g/L) |
| CJ7V:ilvD | 73 | 2.6 |
| CJ7V:ilvD-avtA(del) | 71 | 2.8 |
| CJ7V:ilvD-avtA(a1g) | 73 | 2.6 |
| CJ8V:ilvD | 89 | 1.9 |
| CJ8V:ilvD-avtA(del) | 86 | 2.1 |
| CJ8V:ilvD-avtA(a1g) | 89 | 2.0 |
| KCCM11201P:ilvD | 60 | 2.9 |
| KCCM11201P:ilvD-avtA(del) | 57 | 3.2 |
| KCCM11201P:ilvD-avtA (a1g) | 60 | 2.9 |

As shown in the results above, when the avtA was deleted, the valine-producing ability was enhanced, and when the avtA expression was weakened, the valine-producing ability was increased in the CJ8V:ilvD-avtA (alg) strain compared to the CJ8V:ilvD strain, showing an equal or higher level of valine-producing ability compared the control ilvD-enhanced strains. It was confirmed that the valine-producing ability was enhanced in all cases of ilvD-enhanced strains, and avtA-deleted or weakened strains as compared to the CJ7V, CJ8V and KCCM11201P strains as shown in Table 4.

### Example 2-3. Construction of aceE -Deleted and Weakened (a1g, Q432A, K435A) Strains and Evaluation Thereof

In order to construct strains having high valine-producing ability, an attempt was made to weaken or delete aceE. Accordingly, in order to construct aceE-weakened strains, a vector for constructing a strain, in which the start codon of the aceE gene is modified to GTG, was constructed using primer pairs of SEQ ID NOS: 27 and 28, and SEQ ID NOS: 29 and 30, a vector for constructing aceE(Q432A) strain was constructed using primer pairs of SEQ ID NOS: 27 and 32, and SEQ ID NOS: 33 and 36, and a vector for constructing aceE(K435A) strain was constructed using primer pairs of SEQ ID NOS: 27 and 34, and SEQ ID NOS: 35 and 36. Additionally, a vector for constructing an aceE-deleted strain (aceE(del)) was constructed using primer pairs of SEQ ID NOS: 37 and 38, and SEQ ID NOS: 39 and 40. The thus-constructed vectors were named pDZ-aceE(A1g), pDZ-aceE(Q432A), pDZ-aceE(K435A), and pDZ-aceE(del), respectively.

Specifically, in order to construct the vector for weakening the start codon of aceE, PCR was performed using primer pairs of SEQ ID NOS: 27 and 28, and SEQ ID NOS: 29 and 30, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments obtained above as a template using a primer pair of SEQ ID NOS: 27 and 30 to obtain a mutation-introduced fragment 6. The thus-obtained mutation-introduced fragment 6 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-aceE(A1g) DNA vector.

In order to construct the vector for introducing the aceE(Q432A) mutation, PCR was performed using primer pairs of SEQ ID NOS: 27 and 28, and SEQ ID NOS: 33 and 36, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments obtained above as a template using a primer pair of SEQ ID NOS: 27 and 36 to obtain a mutation-introduced fragment 7. The thus-obtained mutation-introduced fragment 7 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-aceE(Q432A) DNA vector.

In order to construct the vector for introducing the aceE(K435A) mutation, PCR was performed using primer pairs of SEQ ID NOS: 27 and 34, and SEQ ID NOS: 35 and 36, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments obtained above as a template using a primer pair of SEQ ID NOS: 27 and 36 to obtain a mutation-introduced fragment 8. The thus-obtained mutation-introduced fragment 8 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-aceE(K435A) DNA vector.

In order to introduce the vector for introducing the aceE(del) mutation, PCR was performed using primer pairs of SEQ ID NOS: 37 and 38, and SEQ ID NOS: 39 and 40, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments obtained above as a template using a primer pair of SEQ ID NOS: 37 and 40 to obtain a mutation-introduced fragment 9. The thus-obtained mutation-introduced fragment 9 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-aceE(del) DNA vector.

**[Table 7]**

| Primer Sequences for Constructing Vectors of aceE-Deleted and Weakened Strains | | |
|---|---|---|
| Primers | Nucleotide Sequences | SEQ ID NO: |
| Primer 19 | GCTCGGTACCCGGGGATCCTCTAGATAC CGTCCAACCGGTACTTTGAACC | 27 |
| Primer 20 | TTGATCGGCCACTTCCACAC | 28 |
| Primer 21 | GGTGTGGAAGTGGCCGATCAA | 29 |
| Primer 22 | GCCTGCAGGTCGACCTAGATCTAGAGAT CGTCTTCAGAAAGGCGACCCTC | 30 |
| Primer 23 | GCTCGGTACCCGGGGATCCTCTAGAAC CGTGGCATCAAGGACACCTCTGA | 31 |
| Primer 24 | AGCTTCTTCATTGCGTGGGTTG | 32 |
| Primer 25 | CAACCCACGCAATGAAGAAGCT | 33 |
| Primer 26 | AAGCGTCAGTGCCTTCATCTG | 34 |
| Primer 27 | CCAGATGAAGGCACTGACGCTT | 35 |
| Primer 28 | GCCTGCAGGTCGACCTAGATCTAGAAGA TGGAGTCACCGGTGCGCTGGAA | 36 |
| Primer 29 | GCTCGGTACCCGGGGATCCTCTAGAAC CTTTCCCTGGAATTTTTTCCTTT | 37 |
| Primer 30 | TGTCCCTTGAGGTGATTTCCACACCTCC TGTTGGAATG | 38 |
| Primer 31 | TCCAACAGGAGGTGTGGAAATCACCTCA AGGGACAGA | 39 |
| Primer 32 | GCCTGCAGGTCGACCTAGATCTAGATGC TGCGCGGCAAGCGCCGTGGATT | 40 |

Thereafter, the pDZ-aceE(del), pDZ-aceE(A1g), pDZ-aceE(Q432A), and pDZ-aceE(K435A) were transformed into each of the CJ7V:ilvD, CJ8V:ilvD, and KCCM11201P:ilvD, which are valine-producing strains, to induce homologous recombination on the chromosome (van der Rest et al., Appl Microbiol Biotechnol 52:541-545, 1999). The strains introduced with the vectors on the chromosome by recombination of homologous sequences were selected in a medium containing 25 mg/L of kanamycin.

Subsequently, the gene fragments were amplified based on the *Corynebacterium glutamicum* transformants selected above by PCR using a primer pair of SEQ ID NOS: 27 and 36, and the introduction of the mutation was confirmed by gene sequencing analysis. The recombinant strains were named *Corynebacterium glutamicum* CJ7V:ilvD-aceE(del), CJ7V:ilvD-aceE (a1g), CJ7V:ilvD-aceE(Q432A), CJ7V:ilvD-aceE(K435A), CJ8V:ilvD-aceE(del), CJ8V:ilvD-aceE (a1g), CJ8V:ilvD-aceE(Q432A), CJ8V:ilvD-aceE(K435A), KCCM11201P:ilvD-aceE(del), KCCM11201P:iIvD-aceE(a1g), KCCM11201P:ilvD-aceE(Q432A), and KCCM11201P:ilvD-aceE(K435A) as shown below, and the titer evaluation was performed in the same manner as in Example 1.

**[Table 8]**

| L-Valine-Producing Ability of aceE-Deleted and Weakened Strains | | |
|---|---|---|
| Strains | OD600 | Valine (g/L) |
| CJ7V:ilvD | 73 | 2.6 |
| CJ7V:ilvD-aceE-deleted | 21 | 1.1 |
| CJ7V:ilvD-aceE-weakened(a1g) | 69 | 2.8 |
| CJ7V:ilvD-aceE-weakened (Q432A) | 59 | 2.7 |
| CJ7V:ilvD-aceE-weakened (K435A) | 62 | 3.0 |
| CJ8V:ilvD | 89 | 1.9 |
| CJ8V:ilvD-aceE-deleted | 29 | 1.1 |
| CJ8V:ilvD-aceE-weakened (a1g) | 87 | 2.0 |
| CJ8V:ilvD-aceE-weakened (Q432A) | 77 | 2.1 |
| CJ8V:ilvD-aceE-weakened (K435A) | 81 | 2.4 |
| KCCM11201P:ilvD | 60 | 2.9 |
| KCCM11201P:ilvD-aceE-deleted | 13 | 1.0 |
| KCCM11201P:ilvD-aceE-weakened (a1g) | 59 | 3.0 |
| KCCM11201P:ilvD-aceE-weakened (Q432A) | 43 | 2.9 |
| KCCM11201P:ilvD-aceE-weakened (K435A) | 53 | 3.4 |

As shown in the results above, when the aceE was additionally deleted in the strains having enhanced activity of dihydroxy-acid dehydratase (ilvD), the growth and sugar consumption rates were rapidly reduced, resulting in reduced valine-producing ability. Meanwhile, in the case of strains with weakened aceE, it was confirmed that the growth and sugar consumption rates were insignificant and the valine-producing ability was increased, although there were differences according to the level of weakening.

### Example 2-4. Construction of gltA-Weakened (a1g, N241T, M312I) Strains and Evaluation Thereof

In order to construct strains having high valine-producing ability, an attempt was made to weaken gltA. Accordingly, a vector for constructing a gltA-weakened strain, in which the start codon of the gltA gene is modified to GTG, was constructed using primer pairs of SEQ ID NOS: 41 and 42, and SEQ ID NOS: 43 and 44, a vector for constructing gltA(N241T) strain was constructed using primer pairs of SEQ ID NOS: 45 and 46, and SEQ ID NOS: 47 and 50, and a vector for constructing gltA(M312I) strain was constructed using primer pairs of SEQ ID NOS: 45 and 48, and SEQ ID NOS: 49 and 50. The thus-constructed vectors were named pDZ-gltA(Alg), pDZ-gltA(N241T), and pDZ-gltA(M312I), respectively.

In order to introduce the vector for introducing the mutation of weakening the gltA start codon, PCR was performed using primer pairs of SEQ ID NOS: 41 and 42, and SEQ ID NOS: 43 and 44, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments (A, B) obtained above as a template using a primer pair of SEQ ID NOS: 41 and 44 to obtain a mutation-introduced fragment 10. The thus-obtained mutation-introduced fragment 10 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-gltA(Alg) DNA vector.

In order to introduce the vector for introducing the gltA(N241T) mutation, PCR was performed using primer pairs of SEQ ID NOS: 45 and 46, and SEQ ID NOS: 47 and 50, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments (A, B) obtained above as a template using a primer pair of SEQ ID NOS: 45 and 50 to obtain a mutation-introduced fragment 11. The thus-obtained mutation-introduced fragment 11 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-gltA(N241T) DNA vector.

In order to introduce the vector for introducing the gltA(M312I) mutation, PCR was performed using primer pairs of SEQ ID NOS: 45 and 48, and SEQ ID NOS: 49 and 50, based on the genomic DNA of the wild-type *Corynebacterium glutamicum* ATCC14067 as a template. PCR conditions are as follows: denaturation at 94°C for 5 minutes, and then 25 cycles of denaturation at 94°C for 30 seconds; annealing at 55°C for 30 seconds; and polymerization at 72°C for 60 seconds, followed by polymerization at 72°C for 7 minutes. Overlapping PCR was performed based on each of the fragments (A, B) obtained above as a template using a primer pair of SEQ ID NOS: 45 and 50 to obtain a mutation-introduced fragment 12. The thus-obtained mutation-introduced fragment 12 was treated with Xbal restriction enzyme (New England Biolabs, Beverly, MaA), and then ligated into a pDZ vector, which had been treated with the same restriction enzyme, using T4 ligase (New England Biolabs, Beverly, MA) to construct the pDZ-gltA(M312I) DNA vector.

**[Table 9]**

| Primer Sequences for Constructing Vectors of gltA-Weakened Strains | | |
|---|---|---|
| Primers | Nucleotide Sequences | SEQ ID NO: |
| Primer 37 | GCTCGGTACCCGGGGATCCTCTAGAACC CTGAAGCTGTCAGTTCCTAGCA | 41 |
| Primer 38 | CCCTTTCAAACACATTTGTTC | 42 |
| Primer 39 | CGAACAAATGTGTTTGAAAGGG | 43 |
| Primer 40 | GCCTGCAGGTCGACCTAGATCTAGATGC GCGGTGTGCGTACGCAGCCAGC | 44 |
| Primer 41 | GCTCGGTACCCGGGGATCCTCTAGATAT GTGAGCACTGGCTCTACCGAGT | 45 |
| Primer 42 | AGGTGGAGCAGGTCTGCTCGTG | 46 |
| Primer 43 | CACGAGCAGACCTGCTCCACCT | 47 |
| Primer 44 | TGTCCGAAGCCGATGAGGCGGAC | 48 |
| Primer 45 | CGTCCGCCTCATCGGCTTCGGACA | 49 |
| Primer 46 | GCCTGCAGGTCGACCTAGATCTAGAGTC AGAGATTACGAGATCTTCCGGT | 50 |

Thereafter, the pDZ-gltA(A1g), pDZ-gltA(N241T), and pDZ-gltA(M312I) were transformed into each of the CJ7V:ilvD, CJ8V:ilvD, and KCCM11201 P:ilvD, which are valine-producing strains, to induce homologous recombination on the chromosome (van der Rest et al., Appl Microbiol Biotechnol 52:541-545, 1999). The strains introduced with the vectors on the chromosome by recombination of homologous sequences were selected in a medium containing 25 mg/L of kanamycin.

Subsequently, the gene fragments were amplified based on the *Corynebacterium glutamicum* transformants selected above by PCR using a primer pair of SEQ ID NOS: 45 and 50, and the introduction of the mutation was confirmed by gene sequencing analysis. The recombinant strains were named *Corynebacterium glutamicum* CJ7V:ilvD-gltA (a1g), CJ7V:ilvD-gltA(N241T), CJ7V:ilvD-gltA(M312I), CJ8V:ilvD-gltA(a1g), CJ8V:ilvD-gltA(N241T), CJ8V:ilvD-gltA(M312I), KCCM11201 P:ilvD-gltA(a1g), KCCM11201P:ilvD-gltA(N241T), and KCCM11201P:ilvD-gltA(M312I), and the titer evaluation was performed in the same manner as in Example 1.

**[Table 10]**

| L-Valine-Producing Ability of gltA-Weakened Strains | | |
|---|---|---|
| Strains | OD600 | Valine (g/L) |
| CJ7V:ilvD | 73 | 2.6 |
| CJ7V:ilvD-gltA(a1g) | 69 | 2.7 |
| CJ7V:ilvD-gltA(N241T) | 61 | 2.8 |
| CJ7V:ilvD-gltA(M312I) | 63 | 2.8 |
| CJ8V:ilvD | 89 | 1.9 |
| CJ8V:ilvD-gltA(a1g) | 87 | 1.9 |
| CJ8V:ilvD-gltA(N241T) | 80 | 2.2 |
| CJ8V:ilvD-gltA(M312I) | 81 | 2.1 |
| KCCM11201P:ilvD | 60 | 2.9 |
| KCCM11201P:ilvD-gltA(a1g) | 59 | 3.0 |
| KCCM11201P:ilvD-gltA(N241T) | 51 | 3.2 |
| KCCM11201P:ilvD-gltA(M312I) | 55 | 3.1 |

As shown in the results above, when the gltA was weakened in the strains having enhanced activity of dihydroxy-acid dehydratase (ilvD), it was confirmed that the growth and sugar consumption rates were insignificant and the valine-producing ability was increased, although there were differences according to the level of weakening.

### Example 2-5. Construction of Combination of Strains for Effective Mutant Traits and Evaluation Thereof

From the results confirmed in Examples 2-2 to 2-4, it was attempted to determine whether there was a synergistic effect on the valine-producing ability when various mutations were combined. The pDZ-avtA(del) and pDZ-aceE(K435A) vectors were transformed into each of the CJ7V:ilvD-gltA-weakened(N241T), CJ8V:ilvD-gltA-weakened(N241T), and KCCM11201 P:ilvD-gltA-weakened(N241T), which are valine-producing strains constructed in Example 2-4 (van der Rest et al., Appl Microbiol Biotechnol 52:541-545, 1999). The strains introduced with the vectors on the chromosome by recombination of homologous sequences were selected in a medium containing 25 mg/L of kanamycin.

Thereafter, the gene fragments were amplified based on the *Corynebacterium glutamicum* transformants, in which the secondary recombination was completed, by PCR using primer pairs of SEQ ID NOS: 23 and 26, and SEQ ID NOS: 27 and 36, and then the mutation-introduced strains were confirmed by gene sequencing analysis. The recombinant strains were named as shown below, and the titer evaluation was carried out in the same manner as in Example 1.

**[Table 11]**

| L-Valine-Producing Ability of Effective Combination of Strains | | |
|---|---|---|
| Strains | OD600 | Valine (g/L) |
| CJ7V:ilvD-gltA-weakened(N241T) | 61 | 2.8 |
| CJ7V:ilvD-gltA-weakened(N241T)-aceE-weakened(K435A) | 59 | 3.2 |
| CJ7V:ilvD-gltA-weakened(N241T)-avtA-deleted(del) | 62 | 3.0 |
| CJ8V:ilvD-gltA-weakened(N241T) | 80 | 2.2 |
| CJ8V:ilvD-gltA-weakened(N241T) aceE-weakened(K435A) | 78 | 2.6 |
| CJ8V:ilvD-gltA-weakened(N241T) avtA-deleted (del) | 83 | 2.5 |
| KCCM11201P:ilvD-gltA-weakened(N241T) | 51 | 3.2 |
| KCCM11201P:ilvD-gltA-weakened(N241T) aceE-weakened(K435A) | 50 | 3.6 |
| KCCM11201P:ilvD-gltA-weakened(N241T) avtA-deleted(del) | 52 | 3.6 |

As shown in the results above, it was confirmed that when the aceE-weakened strains and the avtA-deleted strains were introduced into the ilvD-enhanced and gltA-weakened strains, the growth and sugar consumption rates were at the same level, and the valine-producing ability was further increased.

The pDZ-aceE(K435A) vector was transformed into the KCCM1 1201P:ilvD-gltA-weakened (N241T) strain, and the strain introduced with the ace(K435A) mutation on the chromosome by recombination of homologous sequences was named CA08-1592. CA08-1592 was deposited at the Korean Culture Center of Microorganisms (KCCM) under Budapest Treaty on July 3, 2020, with Accession No. KCCM12761P.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

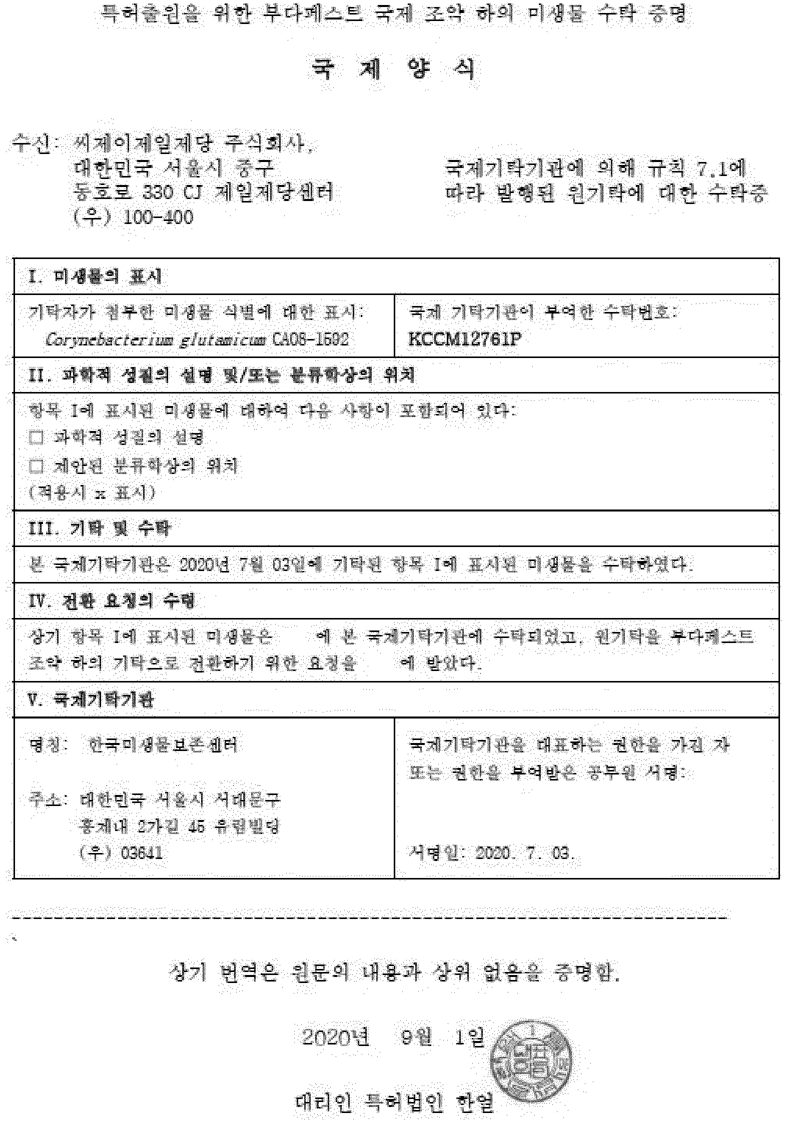
특허출원을 위한 부다페스트 국제 조약 하의 미생물 수탁 증명

국 제 양 식

수신: 씨제이제일제당 주식회사,
대한민국 서울시 중구 | 국제기탁기관에 의해 규칙 7.1에
동호로 330 CJ 제일제당센터 | 따라 발행된 원기탁에 대한 수탁증
(우) 100-400

I. 미생물의 표시
기탁자가 첨부한 미생물 식별에 대한 표시:
Corynebacterium glutamicum CA08-1592 | 국제 기탁기관이 부여한 수탁번호:
KCCM12761P
II. 과학적 성질의 설명 및/또는 분류학상의 위치
항목 I에 표시된 미생물에 대하여 다음 사항이 포함되어 있다:
□ 과학적 성질의 설명
□ 제안된 분류학상의 위치
(적용시 x 표시)
III. 기탁 및 수탁
본 국제기탁기관은 2020년 7월 03일에 기탁된 항목 I에 표시된 미생물을 수탁하였다.
IV. 전환 요청의 수령
상기 항목 I에 표시된 미생물은 에 본 국제기탁기관에 수탁되었고, 원기탁을 부다페스트
조약 하의 기탁으로 전환하기 위한 요청을 에 받았다.
V. 국제기탁기관
명칭: 한국미생물보존센터

주소: 대한민국 서울시 서대문구
홍제내 2가길 45 유림빌딩
(우) 03641 | 국제기탁기관을 대표하는 권한을 가진 자
또는 권한을 부여받은 공무원 서명:

서명일: 2020. 7. 03.

상기 번역은 원문의 내용과 상위 없음을 증명함.

2020년 9월 1일

대리인 특허법인 한얼

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA08-1592 | Accession number given by the
INTERNATIONAL DEPOSITARY AUTHORITY:
KCCM12761P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on July. 03. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: July. 03. 2020.

¹ Where Rule 6.4(d) applies, such date is the date on which the status of international depositary authority was
acquired.

Form BP/4 (sole page)

## Claims

1. A microorganism producing L-valine having enhanced dihydroxy-acid dehydratase activity; and any one or more combinations selected from (1) to (3) below:
(1) reduced transaminase C activity;
(2) weakened pyruvate dehydrogenase activity; and
(3) reduced citrate synthase activity.

2. The microorganism of claim 1, wherein the microorganism has enhanced dihydroxy-acid dehydratase activity; and reduced transaminase C activity.

3. The microorganism of claim 1, wherein the microorganism has enhanced dihydroxy-acid dehydratase activity; and weakened pyruvate dehydrogenase activity.

4. The microorganism of claim 1, wherein the microorganism has enhanced dihydroxy-acid dehydratase activity; and reduced citrate synthase activity.

5. The microorganism of claim 4, wherein the microorganism further has reduced transaminase C activity or weakened pyruvate dehydrogenase activity.

6. The microorganism of claim 1, wherein the dihydroxy-acid dehydratase is encoded by the ilvD gene.

7. The microorganism of claim 1, wherein the transaminase C is encoded by the avtA gene.

8. The microorganism of claim 1, wherein the pyruvate dehydrogenase is encoded by the aceE gene.

9. The microorganism of claim 1, wherein the citrate synthase is encoded by the gltA gene.

10. The microorganism of claim 1, wherein the weakened pyruvate dehydrogenase is **characterized in that** the amino acid corresponding to position 432 or 435 from the N-terminus of the amino acid sequence of SEQ ID NO: 3 is substituted with another amino acid.

11. The microorganism of claim 1, wherein the reduced citrate synthase is **characterized in that** the amino acid corresponding to position 241 or 312 from the N-terminus of the amino acid sequence of SEQ ID NO: 4 is substituted with another amino acid.

12. The microorganism of claim 1, wherein the microorganism producing L-valine is a microorganism of the genus *Corynebacterium.*

13. The microorganism of claim 12, wherein the microorganism of the genus *Corynebacterium* is *Corynebacterium glutamicum.*

14. A method for producing L-valine, comprising culturing the microorganism of any one of claims 1 to 13.

15. The method of claim 14, wherein the method further comprises recovering L-valine from the cultured microorganism or medium.
